# FASCICULE DE BREVET EUROPEEN

(11) **EP 1 813 266 B1**
(45) Date de publication et mention de la délivrance du brevet: **26.06.2013**
(21) Numéro de dépôt: 06126913.0
(22) Date de dépôt: 21.12.2006
(51) Int. Cl.: A61K 8/88, A61Q 17/04

(54) **Composition photoprotectrice fluide aqueuse a base d'un polymere poly(ester-amide) a terminaison ester**
Wässrige flüssige lichtschützende Zusammensetzung auf Basis von Polyesteramiden mit Ester-Endgruppen
Aqueous fluid photoprotective composition based on an ester-terminated poly(ester amide) polymer

(30) Priorité: 19.01.2006 FR 0650192
(43) Date de publication de la demande: 01.08.2007
(73) Titulaire: L'Oréal, 75008 Paris (FR)
(72) Inventeur: Josso, Martin, 75007 Paris (FR); Chevalier, Cyril, 91260 Juvisy-sur-Orge (FR); Gaudry, Anne-Laure, 75013 Paris (FR)
(74) Mandataire: Miszputen, Laurent

(56) Documents cités:
- EP-A2- 1 421 931
- WO-A-02/092663
- WO-A-2006/001940
- US-A- 6 139 827
- US-A1- 2004 247 549
- US-A1- 2005 197 479

## Description

La présente invention concerne une composition fluide, destinée à la protection de la peau et/ou des cheveux contre le rayonnement ultraviolet, caractérisée par le fait qu'elle comprend, dans un support aqueux cosmétiquement acceptable au moins :
(a) un système photoprotecteur capable de filtrer le rayonnement UV ;
(b) un polymère poly(ester-amide) à terminaison ester (ETPEA) particulier.

La présente invention concerne plus particulièrement des compositions fluides vaporisables aqueuses notamment sous forme de sprays comprenant l'association de :
(a) au moins un système photoprotecteur capable de filtrer le rayonnement UV ;
(b) au moins un polymère poly(ester-amide) à terminaison ester (ETPEA) particulier.

Elle concerne également un dispositif comprenant au moins (A) un réservoir contenant au moins une composition aqueuse fluide vaporisable telle que définie ci-dessus et (B) des moyens permettant de mettre sous pression ladite composition en particulier du type pompe non-aérosol (atomiseur), ou du type aérosol ou pompe aérosol.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.
De nombreuses compositions cosmétiques destinées à la photoprotection (UV-A et/ou UV-B) de la peau ont été proposées à ce jour. On recherche tout particulièrement des formulations fluides qui assurent pour les utilisateurs une application facile sur la peau.

Ces compositions fluides anti-solaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV.

Aussi, il existe un besoin croissant de produits solaires fluides ayant un indice de protection élevé. Les indices de protection élevés peuvent être atteints en incorporant plus de filtres à des concentrations élevées. Ceci n'est pas toujours réalisable malgré l'addition de quantités importantes de filtres De plus de telles quantités peuvent entraîner des désagréments au niveau du confort (effet collant, rèche et/ou effet gras).

Les produits solaires présentés sous forme de spray sont de plus en plus recherchés par les consommateurs, à cause de leur facilité d'utilisation et de leur agrément cosmétique.

Pour répondre à cet objectif, on a déjà préconisé, dans la demande EP1421931, d'utiliser des microparticules sphériques de silice poreuse. Cependant, les sprays ainsi obtenus ont tendance dans certains cas à produire sur la peau après application un peluchage.

On connaît dans la demande de brevet US2003/0236387 des compositions cosmétiques notamment des formulations de maquillage sous forme de gel ou de composition solide comprenant au moins un polymère poly(ester-amide) à terminaison ester (ETPEA) dans lesquelles ledit polymère est présent comme agent gélifiant ou structurant.

On connaît dans la demande de brevet W02006/001940 des émulsions solaires comprenant comme polymère poly(ester-amide) à terminaison ester (ETPEA), le polymère poly(ester-amide) à terminaison ester Bis-Stearyl Ethylenediamine/Neopentyl Glycol/Stearyl Hydrogenated Dimer Dilinoleate Copolymer (nom INCI) particulier dans lesquelles ledit polymère est utilisé comme agent améliorant la rémanence à l'eau et permettant de diminuer la migration du produit à travers la peau de l'utilisateur et de limiter les performances de glissement-adhérence.

Or, à la suite d'importantes recherches menées dans le domaine de la photoprotection évoqué ci-dessus, la Demanderesse a découvert de manière surprenante que l'utilisation d'un polymère poly(ester-amide) à terminaison ester (ETPEA) dans une composition aqueuse fluide contenant au moins un système filtrant les radiations UV, permettait d'obtenir une composition antisolaire fluide ayant des indices de protection supérieurs à ceux qui peuvent être obtenus avec le même système photoprotecteur seul et en particulier des sprays à indice élevé sans les inconvénients énoncés ci-dessus.

Par « composition fluide », on entend au sens de l'invention une composition ne se présentant pas sous une forme solide et ont la viscosité mesurée à l'aide d'un viscosimètre Rhéomat 180 à 25°C à la vitesse de rotation de 200 RPM après 30 secondes de rotation est inférieure à 2 Pa.s.

Cette découverte est à la base de la présente invention. Ainsi, conformément à un premier objet de la présente invention, il est proposé de nouvelles compositions fluides destinées à la protection de la peau et/ou des cheveux contre le rayonnement ultraviolet, caractérisées par le fait qu'elles comprennent, dans un support aqueux cosmétiquement acceptable au moins :
(a) un système photoprotecteur capable de filtrer le rayonnement UV ;
(b) le polymère poly(ester-amide) à terminaison ester (ETPEA) Bis-Stearyl Ethylenediamine/Neopentyl Glycol/Stearyl Hydrogenated Dimer Dilinoteate Copolymer ; ladite composition ayant une viscosité inférieure ou égale à 0,5 Pa.s ; ladite viscosité étant mesurée à l'aide d'un viscosimètre Rhéomat 180 à 25°C à la vitesse de rotation de 200 RPM après 30 secondes de rotation.

Selon l'invention, on entend désigner de manière générale par « système photoprotecteur capable de filtrer le rayonnement UV », tout composé ou toute association de composés qui, par des mécanismes connus en soi d'absorption et/ou de réflexion et/ou diffusion du rayonnement UV-A et/ou UV-B, permet d'empêcher, ou du moins limiter, la mise en contact dudit rayonnement avec une surface (peau, cheveux,) sur laquelle ce ou ces composés ont été appliqués. En d'autres termes, ces composés peuvent être des filtres organiques photoprotecteurs absorbeurs d'UV ou des (nano)pigments minéraux diffuseurs et/ou réflecteurs d'UV, ainsi que leurs mélanges.

Par « cosmétiquement acceptable », on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Un autre objet encore de la présente invention réside dans l'utilisation d'au moins polymère poly(ester-amide) à terminaison ester (ETPEA) dans une composition fluide comprenant dans un support aqueux cosmétiquement acceptable au moins un système photoprotecteur capable de filtrer le rayonnement UV, dans le but d'augmenter le facteur de protection solaire (SPF).

Un autre objet encore de la présente invention réside dans l'utilisation d'au moins polymère poly(ester-amide) à terminaison ester (ETPEA) dans une composition fluide comprenant dans un support aqueux cosmétiquement acceptable au moins un système photoprotecteur capable de filtrer le rayonnement UV, dans le but de diminuer voire supprimer l'effet de peluchage.
D'autres caractéristiques, aspects et avantages de la présente invention apparaîtront à la lecture de la description détaillée qui va suivre.
Les polymères poly(ester-amide) à terminaison ester (ETPEA) conformes à l'invention se présentent de préférence sous la forme d'une résine préparée en faisant réagir un dimère de l'acide linoléique hydrogéné en C₃₆, l'éthylènediamine, le néopentylglycol et l'alcool stéarylique dans laquelle :
(i) au moins 50 équivalent % dudit diacide comprend un acide gras polymérisé et
(ii) au moins 50 équivalent % de ladite diamine comprend de l'éthylènediamine.

Plus préférentiellement, la composition de résine est telle que
(iii) 10 à 60 équivalent % par rapport à la totalité des équivalents en hydroxyle et en amine provenant de la diamine, du polyol et du monoalcool proviennent du monoalcool
(iv) au plus 50 équivalent % par rapport à la totalité des équivalents en hydroxyle et en amine provenant de la diamine, du polyol et du monoalcool proviennent du polyol

Les polymères de poly(ester-amide) à terminaison ester (ETPEA) conformes à l'invention peuvent être préparés selon le procédé décrit dans le brevet US 6552160.

Le dimère de l'acide linoléique hydrogéné en C₃₆ de l'invention est hydrogéné avant d'être utilisé dans la réaction de formation de la résine. L'hydrogénation permet d'obtenir un point de fusion de la résine légèrement plus élevé ainsi qu'une plus grande stabilité à l'oxydation et de la couleur dans le cas d'une résine légèrement colorée.

Le réactif diamine est l'éthylène diamine

Le monoalcool réactif **est** l'octadécanol-1 appelé plus communément alcool stéarylique.

Un dernier ingrédient nécessaire à la préparation de la résine d' ETPEA est le néopentylglycol.

Les équivalents en hydroxyle provenant du polyol sont de préférence inférieurs ou égaux à 50% par rapport à la totalité des équivalents en hydroxyle et en amine apportés par les réactifs polyol, monoalcool et diamine. Selon un mode particulier de l'invention, les équivalents en hydroxyle provenant du polyol peuvent être inférieurs ou égaux à 40%, ou inférieurs ou égaux à 30% ou encore inférieurs ou égaux à 20% par rapport à la totalité des équivalents en hydroxyle et en amine apportés par les réactifs polyol, monoalcool et diamine.

Les équivalents en amine de préférence varient de 0,3 à 0,75 par rapport à la totalité des équivalents en hydroxyle et en amine apportés par les réactifs polyol, monoalcool et diamine. Selon un mode particulier de l'invention, les équivalents en hydroxyle provenant du polyol varient de 0,05 à 0.45 par rapport à la totalité des équivalents en hydroxyle et en amine apportés par les réactifs polyol, monoalcool et diamine. Selon un mode particulier de l'invention, les équivalents en hydroxyle provenant du monoalcool varient de 0,20 à 0,45 par rapport à la totalité des équivalents en hydroxyle et en amine apportés par les réactifs polyol, monoalcool et diamine.

On utilisera plus particulièrement le polymère poly(ester-amide) à terminaison ester Bis-Stearyl Ethylenediamine/Neopentyl Glycol/Stearyl Hydrogenated Dimer Dilinoleate Copolymer (nom INCl) qui est un copolymère de diacide linoléique hydrogéné, d'éthylènediamine, de néopentylglycol et d'alcool stéarylique. Ce copolymère est notamment vendu sous le nom commercial SYLVACLEAR C75 V par la société ARIZONA CHEMICAL.

Le polymère poly(ester-amide) à terminaison ester conforme à l'invention est présent dans la composition de préférence dans une quantité maximale de 10% en poids, plus préférentiellement de 0.1 à 10% en poids et encore plus préférentiellement de 0,5 à 5% et encore plus particulièrement de 1 à 3% par rapport au poids total de la composition.

Selon l'invention, le système photoprotecteur peut être constitué par un ou plusieurs filtres organiques hydrophiles, lipophiles ou insolubles et/ou un ou plusieurs (nano)pigments minéraux. De manière préférentiel, il sera constitué d'au moins un filtre UV organique hydrophile, lipophile ou insoluble.

Les filtres UV organiques hydrophiles, lipophiles ou insolubles sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial
« PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β ,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
la 2,4,6-tris-(4'-amino benzalmalonate de diisobutyle)-s- triazine.

### Dérivés anthraniliques :

Menthyl anthranilate vendu sous le nom commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les agents filtrant les radiations UV organiques préférentiels sont choisis parmi
Ethylhexyl Methoxycinnamate
Homosalate
Ethylhexyl Salicylate,
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine.
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les filtres inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.
Les silicones utilisées pour l'enrobage des nanopigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydrogénosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA et le produit " Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO₂ traité par un polydiméthylsiloxane et dont la taille moyenne des particules élémentaires est de 21 nm tel que celui vendu sous la dénomination commerciale "70250 CARDRE UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-COTE" par la société SUNSMART;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les pigments d'oxyde de zinc enrobés sont par exemple
- ceux commercialisés sous la dénomination « Z-COTE HP1 » par la société SUNSMART (ZnO enrobé dimethicone) ;
- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en C₁₂-C₁₅) ;
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et polymethylsilsesquioxane) ;
- ceux commercialisés sous la dénomination "NANOX GEL TN" par la société Elementis (ZnO dispersé à 55% dans du benzoate d'alcools en C₁₂-C₁₅ avec polycondensat d'acide hydroxystéarique).

Les pigments d'oxyde de cérium non enrobé sont vendus par exemple sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".
Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Le système photoprotecteur selon l'invention est de préférence présent dans les compositions selon l'invention à une teneur allant de 0,1 % à 40 % en poids et en particulier de 5 à 25 % , en poids, par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « ELDEW SL-205 » par la société AJINOMOTO), les acides ou les esters gras (comme le benzoate d'alcools en C₁₂-C₁₅ vendu sous la dénomination commerciale « FINSOLV TN » ou « WITCONOL TN » par la société WITCO, le palmitate d'octyle, le lanolate d'isopropyle, les triglycérides dont ceux des acides caprique/caprylique, le dicaprylyl carbonate vendu sous la dénomination « CETIOL CC » par la société COGNIS), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée, les cires de polyéthylène et les cires de polyméthylène comme celle vendue sous la dénomination CIREBELLE 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C₁₀-C₃₀-alkylacrylate) ; les polyacrylamides comme par exemple les copolymères réticulés vendus sous les noms SEPIGEI 305 (nom C.T.F.A. : polyacrylamide/C13-14 isoparaffin/Laureth 7) ou SIMULGEL 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « HOSTACERIN AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ; et leurs mélanges.

Comme épaississants lipophiles, on peut citer les polymères synthétiques tels que les poly C₁₀-C₃₀ alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Parmi les actifs, on peut citer :
- les vitamines (A, C, E, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société DOW CORNING, et les alkyldimethicone copolyols tels que le Laurylmethicone copolyol vendu sous la dénomination "DOW CORNING 5200 FORMULATION AID" par la société DOW CORNING ; le Cetyl dimethicone copolyol tel que le produit vendu sous la dénomination Abil EM 90R par la société GOLDSCHMIDT et le mélange de cétyl diméthicone copolyol, d'isostéarate de polyglycérole (4 moles) et de laurate d'hexyle vendu sous la dénomination ABIL WE 09 par la société GOLDSCHMIDT. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom ARLACEL P135 par la socité ICI;

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination ARLACEL 987 par la société ICI ; l'isostéarate de sorbitan et le glycérol, tel que le produit commercialisé sous la dénomination ARLACEL 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination ARLACEL 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives PLANTAREN 2000 et PLANTAREN 1200, le cétostéarylglucoside éventuellement en mélange avec l'alcool cétostéarylique, commercialisé par exemple sous la dénomination MONTANOV 68 par la société SEPPIC, sous la dénomination TEGOCARE CG90 par la société Goldschmidt et sous la dénomination EMULGADE KE3302 par la société Henkel, ainsi que l'arachidyl glucoside, par exemple sous la forme du mélange d'alcools arachidique et béhénique et d'arachidylglucoside commercialisé sous la dénomination MONTANOV 202 par la société SEPPIC. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / Isophtalate / 1 ,4-cyclohexane-diméthanol (nom INCI : Polyester-5) vendu sous les dénominations "EASTMAN AQ POLYMER" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Selon une forme préférée, la viscosité des compositions mesurée à l'aide d'un viscosimètre Rhéomat 180 à 25°C à la vitesse de rotation de 200 RPM après 30 secondes de rotation est inférieure ou égale à 0,5 Pa.s.

Selon une forme particulièrement préférée, les compositions selon l'invention se présentent sous forme fluide vaporisable appliquée sur la peau ou les cheveux sous forme de fines particules au moyen de dispositifs de pressurisation.

Selon l'invention, on entend désigner de manière générale par "composition vaporisable", toute composition susceptible de produire sous pression dans un dispositif approprié de fines particules.

La présente invention concerne également un dispositif de pressurisation comprenant au moins (A) un réservoir contenant au moins une composition fluide vaporisable comprenant, dans un support aqueux cosmétiquement acceptable au moins :
(a) un système photoprotecteur capable de filtrer le rayonnement UV tel que défini précédemment ;
(b) un polymère poly(ester-amide) à terminaison ester (ETPEA) tel que défini précédemment ;
et (B) des moyens permettant de mettre sous pression ladite composition.

Les dispositifs conformes à l'invention sont bien connus de l'homme de l'art et comprennent les pompes non-aérosols ou "atomiseurs", les récipients aérosols mono ou bi-compartimentés comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol mono-compartimentés conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les aérosols bi-compartimentés sont pourvus d'une poche dans laquelle se trouve la composition conforme à l'invention. L'agent propulseur se trouve dans le bidon et à l'extérieur de la poche. Il reste à l'intérieur du dispositif durant l'utilisation et exerce une pression sur la poche. Cet agent propulseur peut être un gaz liquéfié tel les agents propulseurs utilisés dans les aérosols mono-compartimentés mais également un gaz comprimé comme l'air ou l'azote.

Les exemples concrets, mais nullement limitatifs, illustrant l'invention, vont maintenant être donnés. L'invention va maintenant être décrite en référence aux exemples suivants donnés à titre illustratif et non limitatif.

Dans ces exemples, sauf indication contraire, les quantités sont exprimées en pourcentages pondéraux.

On a réalisé les formulations solaires suivantes ; les quantités sont indiquées en pourcentages en poids :

### EXEMPLES 1 et 2 :

On a préparé des formulations antisolaires fluides vaporisables contenant les ingrédients suivants.

| **Ingrédients** | **Ex1** | **Ex2** |
|---|---|---|
| Ethylhexyl Salicylate (NEO HELIOPAN OS) | 5,0 | 5,0 |
| Butyl Methoxy Dibenzoylméthane (PARSOL 1789 - DSM) | 4,0 | 4,0 |
| Octocrylene (UVINUL N539 - BASF) | 3,5 | 3,5 |
| Bis-Ethylhexyloxyphenol Methoxyphenyl Triazine (TINOSORB S-CIBA) | 3,0 | 3,0 |
| Ethylhexyl Triazone (UVINUL T150- BASF) | 2,5 | 2,5 |
| Terephthalylidene Dicamphor Sulfonic Acid (MEXORYL SX-CHIMEX) | 1,0 | 3,0 |
| Drometrizole Trisiloxane (MEXORYL X-CHIMEX) | 1,5 | 1,5 |
| Dioxyde de Titane (MICRO TITANIUM DIOXIDE MT100 AQ - TAYCA) | 3,5 | 3,5 |
| Alcool éthylique dénaturé | 4,3 | 4,3 |
| Benzoate d'alkyle en C12-C15 | 12,5 | 12,5 |
| Glycérine | 6 | 6 |
| Tocopherol (and) Glycin Soja | 0,2 | 0,2 |
| Sel pentasodique de l'acide éthylènediamine tétraméthylène phosphonique dans l'eau à 33% (DEQUEST 2046 - SOLUTIA) | 0,3 | 0,3 |
| Propylène glycol | 6,0 | 6,0 |
| Ethylhexyl Glycerin (SENSITIVA SC 50 - SCHULKE & MAYR) | 0,5 | 0,5 |
| Polyester-5 (EASTMAN AQ 38S -EASTMAN CHEMICAL) | 2,0 | 2,0 |
| Bis-Stearyl Ethylenediamine/Neopentyl Glycol/Stearyl Hydrogenated Dimer Dilinoleate Copolymer (SYLVACLEAR C75 V- ARIZONA CHEMICAL) | 1,5 | - |
| Polyacrylate-3 en émulsion dans l'eau à 25% (VISCOPHOBE DB 1000- AMERCHOL) | 0,7 | 0,7 |
| Triéthanolamine | 0,7 | 0,66 |
| Eau déminéralisée | qsp 100 | qsp 100 |

La composition ne peluche pas à l'application sur la peau.

On a ensuite préparé une formulation antisolaire 2 comparative de même composition que la formulation A mais ne contenant pas de polymère ETPEA.

Les viscosités des compositions 1 et 2 mesurées à l'aide d'un viscosimètre Rhéomat 180 à 25°C à la vitesse de rotation de 200 RPM au bout de 30 secondes sont respectivement 0,32 Pa.s et 0,27 Pa.s

Pour chacune des compositions 1 et 2, on a ensuite déterminé le facteur de protection solaire (SPF) qui lui était attaché. Celui-ci a été déterminé en utilisant la méthode in vitro décrite par B. L. DIFFEY et al. Dans J. Soc. Cosmet. Chem. 40 127-133 (1989) ; cette méthode consiste à déterminer les facteurs de protection monochromatiques dans une gamme de longueurs d'onde de 290 à 400 nm et à calculer à partir de ceux-ci le facteur de protection solaire selon une équation mathématique donnée. La mesure a été réalisée avec un pas de 1 nm sur un appareil UV-1000S de la société Labsphere, 0,6 mg/cm² de produit étant étalé sur une plaque de PMMA dépolie. Les résultats (valeur moyenne correspondant 5 plaques par produit, 8 points par plaque) sont regroupés dans le tableau (1) ci-dessous :

**Tableau (I)**

| **Composition** | **1 (invention) avec polymère ETPEA** | **2 (hors invention) sans polymère ETPEA** |
|---|---|---|
| SPF moyen (écart type) | 101,9 (16,2) | 64,4 (9,7) |

### EXEMPLES 3 et 4 (hors invention) :

On a préparé la composition 3 antisolaire de l'exemple 2 de la demande WO2006/001940) contenant le polymère Bis-Stearyl Ethylenediamine/Neopentyl Glycol/Stearyl Hydrogenated Dimer Dilinoleate Copolymer suivants ainsi qu'une composition 3 contre-type ne contenant pas ledit polymère.

| **Ingrédients** | | **Ex3** | **Ex4** |
|---|---|---|---|
| | Phase A | | |
| Eau désionisée | | 48,05 | 49,55 |
| PEMULEN TR1 | | 0,38 | 0,38 |
| Propyleneglycol | | 5,0 | 5,0 |
| Disodium EDTA | | 0,01 | 0,01 |
| Conservateur | | 1,0 | 1,0 |

| | Phase B | | |
|---|---|---|---|
| Octinoxate | | 7,5 | 7,5 |
| Oxybenzone | | 6,0 | 6,0 |
| Vitamine E, DL alpha tocophérol | | 0,01 | 0,01 |
| Oleth-3 | | 0,2 | 0,2 |
| Octylsalicylate | | 5,0 | 5,0 |
| Homenthyl salicylate | | 13,0 | 13,0 |
| Bis-Stearyl Ethylenediamine/Neopentyl Glycol/Stearyl Hydrogenated Dimer Dilinoleate Copolymer (SYLVACLEAR C75 V-ARIZONA CHEMICAL) | | 1,5 | - |
| Octocrylene | | 2,0 | 2,0 |

| | Phase C | | |
|---|---|---|---|
| Eau désionisée | | 10 | 10 |
| Triéthanolamine | | 0,35 | 0,35 |

| | Phase D | | |
|---|---|---|---|
| Parfum | | qs | qs |

Les viscosités des compositions 3 et 4 ont été mesurées à l'aide d'un viscosimètre Rhéomat 180 à 25°C à la vitesse de rotation de 200 RPM au bout de 30 secondes sont respectivement 2,0 Pa.s et 1,9 Pa.s

Pour chacune des compositions 3 et 4, on a ensuite déterminé le facteur de protection solaire (SPF) qui lui était attaché. Celui-ci a été déterminé en utilisant la méthode in vitro décrite par B. L. DIFFEY et al. Dans J. Soc. Cosmet. Chem. 40 127-133 (1989) ; cette méthode consiste à déterminer les facteurs de protection monochromatiques dans une gamme de longueurs d'onde de 290 à 400 nm et à calculer à partir de ceux-ci le facteur de protection solaire selon une équation mathématique donnée. La mesure a été réalisée avec un pas de 1 nm sur un appareil UV-1000S de la société Labsphere, 0,6 mg/cm² de produit étant étalé sur une plaque de PMMA dépolie.

Les résultats (valeur moyenne correspondant 5 plaques par produit, 8 points par plaque) sont regroupés dans le tableau (I) ci-dessous :

**Tableau (II)**

| **Composition** | **3 (hors invention) avec polymère ETPEA** | **4 (hors invention) sans polymère ETPEA** |
|---|---|---|
| SPF moyen (écart type) | 34,4 (5,7) | 36,4 (4,5) |
| Viscosité | 2 Pa.s | 1,9 Pa.s |

Dans la composition 3, non fluide au sens de l'invention, correspondant à l'exemple 2 du document W02006/001940, on observe que le polymère ETPEA ne permet pas d'augmenter le SPF.

## Revendications

1. Composition fluide destinée à la protection de la peau et/ou des cheveux contre le rayonnement ultraviolet, **caractérisée par le fait qu'**elle comprend, dans un support aqueux cosmétiquement acceptable au moins :
(a) un système photoprotecteur capable de filtrer le rayonnement UV ;
(b) le polymères poly(ester-amide) à terminaison ester (ETPEA) Bis-Stearyl Ethylenediamine/Neopentyl Glycol/Stearyl Hydrogenated Dimer Dilinoleate Copolymer; ladite composition ayant une viscosité inférieure ou égale à 0,5 Pa.s ; ladite viscosité étant mesurée à l'aide d'un viscosimètre Rhéomat 180 à 25°C à la vitesse de rotation de 200 RPM après 30 secondes de rotation.

2. Composition selon la revendication 1, où le polymère poly(ester-amide) à terminaison ester (ETPEA) se présente sous la forme d'une résine préparée en faisant réagir un dimère de l'acide linoléique hydrogéné en C₃₆, l'éthylènediamine, le néopentylglycol et l'alcoolstéarylique dans laquelle :
(i) au moins 50 équivalent % dudit diacide comprend un acide gras polymérisé et
(ii) au moins 50 équivalent % de ladite diamine comprend de l'éthylènediamine.

3. Composition selon la revendication 2, où la résine est telle que :
(iii) 10 à 60 équivalent % par rapport à la totalité des équivalents en hydroxyle et en amine provenant de la diamine, du polyol et du monoalcool proviennent du monoalcool
(iv) au plus 50 équivalent % par rapport à la totalité des équivalents en hydroxyle et en amine provenant de la diamine, du polyol et du monoalcool proviennent du polyol

4. Composition selon l'une quelconque des revendications 1 à 3, où le polymères poly(ester-amide) à terminaison ester est présent dans la composition dans une quantité maximale de 10% en poids par rapport au poids total de la composition.

5. Composition selon la revendication 4, où le polymère poly(ester-amide) à terminaison ester est présent dans la composition dans des quantités allant de préférence de 0,1 à 10 % en poids et plus préférentiellement de 0,5 à 5% et encore plus particulièrement de 1 à 3% par rapport au poids total de la composition.

6. Composition selon l'une quelconque des revendications 1 à 5, où le système photoprotecteur est constitué par un ou plusieurs fltres organiques hydrophiles, lipophiles ou insolubles et/ou un ou plusieurs (nano)pigments minéraux.

7. Composition selon la revendication 6, où le système photoprotecteur est constitué par au moins un filtre organique hydrophile, lipophile ou insoluble.

8. Composition selon la revendication 6 ou 7, où les filtres organiques sont choisis parmi les anthranilates ; les dérivés cinnamiques ; les dérivés de dibenzoylméthane; les dérivés salicyliques, les dérivés du camphre ; les dérivés de triazine; les dérivés de la benzophénone; les dérivés de, β -diphénylacrylate; les dérivés de benzotriazole; les dérivés de benzalmalonate; les dérivés de benzimidazole; les imidazolines; les dérivés bis-benzoazolyle; les dérivés de l'acide p-aminobenzoïque (PABA) ; ; les dérivés de méthylène bis-(hydroxyphényl benzotriazole); les polymères filtres et silicones filtres ; les diméres dérivés d'α-alkylstyrène ; les 4,4-diarylbutadiènes et leurs mélanges.

9. Composition selon la revendication 8 où les filtres organiques sont choisis parmi
Ethylhexyl Methoxycinnamate
Homosalate
Ethylhexyl Salicylate,
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Suffonic Acid,
Disodium Phenyl Dibenzimidazole Tetta-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-aminobenzalmalonate de diisobutyle)-s- triazine.
Méthylène bis-Benzotriazolyl Tetramethylbutylphénol,
Drometrizole Trisiloxane
Polysilicone-15
Di-néopentyl 4'-méthoxybenzalmalonate
1,1-dicarboxy(2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

10. Composition selon la revendication 6, où les pigments ou les nanopigments sont choisis parmi les oxydes métalliques enrobés ou non.

11. Composition selon la revendication 10, où le ou les agents filtrant les radiations UV minéraux sont choisis parmi les nanopigments d'oxyde de titane, de fer, de zinc, de zirconium ou de cérium, enrobés ou non.

12. Composition selon l'une quelconque des revendications 1 à 11, où le système photoprotecteur est présent à une teneur allant de 0,1 % à 40 % en poids et de préférence de 5 à 25 %, en poids, par rapport au poids total de la composition.

13. Composition selon l'une quelconque des revendications 1 à 12, **caractérisée par le fait que** la composition contient en plus au moins un agent autobronzant.

14. Composition selon l'une quelconque des revendications 1 à 13, où la composition contient en outre au moins un adjuvant cosmétique choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

15. Composition selon l'une quelconque des revendications 1 à 14, **caractérisée par le fait qu'**elle se présente en particulier sous forme d'émulsion, simple ou complexe, d'un gel crème; d'un gel aqueux ; d'une lotion ou bien conditionnée en aérosol sous forme de mousse ou de spray ou bien conditionnée en atomiseur.

16. Composition selon l'une quelconque des revendications 1 à 15, **caractérisée par le fait qu'**elle se présente sous la forme d'une émulsion huile-dans-eau ou eau-dans huile.

17. Composition selon la revendication 16, contenant au moins un polymère d'acide isophtatique ou d'acide sulfoisophtalique.

18. Composition selon la revendication 17, où ledit polymère d'acide isophtalique ou d'acide sulfoisophtalique est le Potyester-5.

19. Composition selon l'une quelconque des revendications précédentes, **caractérisée par le fait qu'**elle se présente sous forme vaporisable.

20. Dispositif de pressurisation comprenant au moins (A) un réservoir contenant au moins une composition fluide vaporisable telle que définie dans l'une quelconque des revendications 1 à 19,
et (B) des moyens permettant de mettre sous pression ladite composition.

21. Dispositif selon la revendication 20, **caractérisé par le fait qu'**il s'agit d'une pompe non-aérosol

22. Dispositif selon l'une quelconque des revendications 20 et 21, **caractérisé par le fait qu'**il s'agit d'un récipient aérosol ou d'une pompe aérosol.

23. Dispositif selon l'une quelconque des revendications 20 à 22, **caractérisé par le fait qu'**il s'agit d'un récipient aérosol ou d'une pompe aérosol bi-compartimenté.

24. Utilisation d'un polymère poly(ester-amide) à terminaison ester tel que défini dans les revendications précédentes dans une composition comprenant dans un support aqueux cosmétiquement acceptable au moins un système photoprotecteur capable de filtrer le rayonnement UV telle que défini dans l'une des revendications précédentes, dans le but d'augmenter le facteur de protection solaire (SPF).

## Patentansprüche

1. Fließfähige Zusammensetzung zum Schutz der Haut und/oder der Haare gegen Ultraviolettstrahlung, **dadurch gekennzeichnet, dass** sie in einem kosmetisch unbedenklichen wässrigen Medium mindestens:
(a) ein Lichtschutzsystem, das zum Filtern von UV-Strahlung befähigt ist;
(b) das esterterminierte Poly(esteramid)-Polymer (ETPEA) Bis-Stearyl Ethylenediamine/Neopentyl Glycol/Stearyl Hydrogenated Dimer Dilinoleate Copolymer
umfasst; wobei die Zusammensetzung eine Viskosität kleiner oder gleich 0,5 Pa.s aufweist; wobei die Viskosität mithilfe eines Rheomat-180-Viskosimeters bei 25°C und einer Rotationsgeschwindigkeit von 200 U/min nach 30 Sekunden Rotation gemessen wird.

2. Zusammensetzung nach Anspruch 1, wobei das esterterminierte Poly(esteramid)-Polymer (ETPEA) in Form eines durch Umsetzung eines durch Umsetzung von einem hydrierten C₃₆-Linolensäuredimer, Ethylendiamin, Neopentylglykol und Stearylalkohol hergestellten Harzes vorliegt, worin:
(i) mindestens 50 Äquivalent-% der Disäure eine polymerisierte Fettsäure umfassen und
(ii) mindestens 50 Äquivalent-% des Diamins Ethylendiamin umfassen.

3. Zusammensetzung nach Anspruch 2, wobei das Harz derart beschaffen ist, dass:
(iii) 10 bis 60 Äquivalent-%, bezogen auf die Gesamtheit der Hydroxyl- und Amin-Äquivalente des Diamins, des Polyols und des Monoalkohols, von dem Monoalkohol stammen,
(iv) höchstens 50 Äquivalent-%, bezogen auf die Gesamtheit der Hydroxyl- und Amin-Äquivalente des Diamins, des Polyols und des Monoalkohols, von dem Polyol stammen.

4. Zusammensetzung nach einem der Ansprüche 1 bis 3, wobei das esterterminierte Poly(esteramid)-Polymer in der Zusammensetzung in einer Höchstmenge von 10 Gew.-%, bezogen auf das Gesamtgewicht der Zusammensetzung, vorliegt.

5. Zusammensetzung nach Anspruch 4, wobei das esterterminierte Poly(esteramid)-Polymer in der Zusammensetzung in Mengen vorliegt, die vorzugsweise im Bereich von 0,1 bis 10 Gew.-% und weiter bevorzugt 0,5 bis 5 Gew.-% und noch weiter bevorzugt 1 bis 3 Gew.-%, bezogen auf das Gesamtgewicht der Zusammensetzung, liegen.

6. Zusammensetzung nach einem der Ansprüche 1 bis 5, wobei das Lichtschutzsystem aus einem oder mehreren hydrophilen, lipophilen oder unlöslichen organischen Filtern und/oder einem oder mehreren anorganischen (Nano)Pigmenten besteht.

7. Zusammensetzung nach Anspruch 6, wobei das Lichtschutzsystem aus mindestens einem hydrophilen, lipophilen oder unlöslichen organischen Filter besteht.

8. Zusammensetzung nach Anspruch 6 oder 7, wobei die organischen Filter aus Anthranilaten; Zimtsäurederivaten; Dibenzoylmethanderivaten; Salicylsäurederivaten; Campherderivaten; Triazinderivaten; Benzophenonderivaten; β-Diphenylacrylatderivaten; Benzotriazolderivaten; Benzalmalonatderivaten; Benzimidazolderivaten; Imidazolinen; Bisbenzoazolylderivaten; p-Aminobenzoesäurederivaten (PABA-Derivaten); Methylenbis-(hydroxyphenylbenzotriazol)derivaten; Filterpolymeren und Filtersilikonen; von α-Alkylstyrol abgeleiteten Dimeren; 4,4-Diarylbutadienen und Mischungen davon ausgewählt sind.

9. Zusammensetzung nach Anspruch 8, wobei die organischen Filter aus
Ethylhexyl Methoxycinnamate
Homosalate
Ethylhexyl Salicylate,
Butyl Methoxydibenzoylmethane,
Octocrylene,
Phenylbenzimidazole Sulfonic Acid,
Benzophenone-3,
Benzophenone-4,
Benzophenone-5,
2-(4-Diethylamino-2-hydroxybenzoyl)benzoesäure-n-hexylester,
4-Methylbenzylidene Camphor,
Terephthalylidene Dicamphor Sulfonic Acid,
Disodium Phenyl Dibenzimidazole Tetra-sulfonate, Ethylhexyl Triazone,
Bis-Ethylhexyloxyphenol Methoxyphenyl Triazine, Diethylhexyl Butamido Triazone,
2,4,6-Tris(dineopentyl-4'-aminobenzalmalonat)-s-triazin,
2,4,6-Tris(diisobutyl-4'-aminobenzalmalonat)-s-triazin,
Methylenbis(benzotriazolyl)tetramethylbutylphenol, Drometrizole Trisiloxane,
Polysilicone-15,
Dineopentyl-4'-methoxybenzalmalonat,
1,1-Dicarboxy(2,2'-dimethylpropyl)-4,4-diphenyl-butadien,
2,4-Bis[5-1(dimethylpropyl)benzoxazol-2-yl(4-phenyl)imino]-6-(2-ethylhexyl)imino-1,3,5-triazin
und Mischungen davon ausgewählt sind.

10. Zusammensetzung nach Anspruch 6, wobei die Pigmente oder Nanopigmente aus beschichteten oder unbeschichteten Metalloxiden ausgewählt sind.

11. Zusammensetzung nach Anspruch 10, wobei das oder die anorganischen UV-Strahlung filtrierenden Mittel aus Nanopigmenten aus Oxiden von Titan, Eisen, Zink, Zirconium oder Cer, die beschichtet oder unbeschichtet sein können, ausgewählt sind.

12. Zusammensetzung nach einem der Ansprüche 1 bis 11, wobei das Lichtschutzsystem in einem Gehalt im Bereich von 0,1 bis 40 Gew.-% und vorzugsweise 5 bis 25 Gew.-%, bezogen auf das Gesamtgewicht der Zusammensetzung, vorliegt.

13. Zusammensetzung nach einem der Ansprüche 1 bis 12, **dadurch gekennzeichnet, dass** die Zusammensetzung außerdem mindestens ein Selbstbräunungsmittel enthält.

14. Zusammensetzung nach einem der Ansprüche 1 bis 13, wobei die Zusammensetzung außerdem mindestens einen kosmetischen Hilfsstoff, der unter Fettsubstanzen, organischen Lösungsmitteln, ionischen oder nichtionischen, hydrophilen oder lipophilen Verdickungsmitteln, zartmachenden Mitteln, Feuchtigkeitsspendern, Trübungsmitteln, Stabilisatoren, Emollientien, Silikonen, Schaumhemmern, Duftstoffen, Konservierungsmitteln, anionischen, kationischen, nichtionischen, zwitterionischen oder amphoteren Tensiden, Wirkstoffen, Füllstoffen, Polymeren, Treibmitteln, Alkalinisierungsmitteln oder Ansäuerungsmitteln oder einem beliebigen anderen Inhaltsstoff, der auf dem Gebiet der Kosmetik und/oder Dermatologie üblicherweise verwendet wird, ausgewählt ist, enthält.

15. Zusammensetzung nach einem der Ansprüche 1 bis 14, **dadurch gekennzeichnet, dass** sie insbesondere in Form einer einfachen oder komplexen Emulsion, eines Cremegels, eines wässrigen Gels, einer Lotion oder als Aerosol in Schaum- oder Sprayform verpackt oder als Zerstäuber verpackt vorliegt.

16. Zusammensetzung nach einem der Ansprüche 1 bis 15, **dadurch gekennzeichnet, dass** sie in Form einer Ölin-Wasser-Emulsion oder Wasser-in-Öl-Emulsion vorliegt.

17. Zusammensetzung nach Anspruch 16, die mindestens ein Isophthalsäure- oder Sulfoisophthalsäurepolymer enthält.

18. Zusammensetzung nach Anspruch 17, wobei es sich bei dem Isophthalsäure- oder Sulfoisophthalsäurepolymer um Polyester-5 handelt.

19. Zusammensetzung nach einem der vorhergehenden Ansprüche, **dadurch gekennzeichnet, dass** sie in verdampfbarer Form vorliegt.

20. Druckbeaufschlagungsvorrichtung, umfassend mindestens (A) einen Behälter, der mindestens eine verdampfbare fließfähige Zusammensetzung gemäß einem der Ansprüche 1 bis 19 enthält,
und (B) Mittel zum Unterdrucksetzen der Zusammensetzung.

21. Vorrichtung nach Anspruch 20, **dadurch gekennzeichnet, dass** es sich um eine Nichtaerosolpumpe handelt.

22. Vorrichtung nach einem der Ansprüche 20 und 21, **dadurch gekennzeichnet, dass** es sich um einen Aerosolbehälter oder eine Aerosolpumpe handelt.

23. Vorrichtung nach einem der Ansprüche 20 bis 22, **dadurch gekennzeichnet, dass** es sich um einen Aerosolbehälter oder eine Aerosolpumpe mit zwei Kompartimenten handelt.

24. Verwendung eines esterterminierten Poly(ester-amid)-Polymers gemäß einem der vorhergehenden Ansprüche in einer Zusammensetzung, die in einem kosmetisch unbedenklichen wässrigen Träger mindestens ein Lichtschutzsystem, das zum Filtern von UV-Strahlung befähigt ist, gemäß einem der vorhergehenden Ansprüche umfasst, zur Erhöhung des Sonnenschutzfaktors (SPF).

## Claims

1. Fluid composition intended for protecting the skin and/or the hair against ultraviolet radiation, **characterized in that** it comprises, in a cosmetically acceptable aqueous support, at least:
(a) one photoprotective system capable of screening out UV radiation;
(b) the ester-terminated poly(ester amide) (ETPEA) polymer Bis-Stearyl Ethylenediamine/Neopentyl Glycol/Stearyl Hydrogenated Dimer Dilinoleate Copolymer; said composition having a viscosity of less than or equal to 0.5 Pa.s; said viscosity being measured using a Rheomat 180 viscometer at 25°C at the rotational speed of 200 rpm after 30 seconds of rotation.

2. Composition according to Claim 1, in which the ester-terminated poly(ester amide) (ETPEA) polymer is in the form of a resin prepared by reacting a C₃₆ hydrogenated linoleic acid dimer, ethylenediamine, neopentyl glycol and stearyl alcohol, in which:
(i) at least 50 equivalent% of said diacid comprises a polymerized fatty acid and
(ii) at least 50 equivalent% of said diamine comprises ethylenediamine.

3. Composition according to Claim 2, in which the resin is such that:
(iii) 10 to 60 equivalent% relative to the total of the equivalents of hydroxyl and of amine originating from the diamine, from the polyol and from the monoalcohol originate from the monoalcohol,
(iv) not more than 50 equivalent% relative to the total of the equivalents of hydroxyl and of amine originating from the diamine, from the polyol and from the monoalcohol originate from the polyol.

4. Composition according to any one of Claims 1 to 3, in which the ester-terminated poly(ester amide) polymer is present in the composition in a maximum amount of 10% by weight relative to the total weight of the composition.

5. Composition according to Claim 4, in which the ester-terminated poly(ester amide) polymer is present in the composition in amounts preferably ranging from 0.1% to 10% by weight, more preferentially from 0.5% to 5% and even more particularly from 1% to 3% relative to the total weight of the composition.

6. Composition according to any one of Claims 1 to 5, in which the photoprotective system is constituted of one or more hydrophilic, lipophilic or insoluble organic screening agents and/or one or more mineral (nano)pigments.

7. Composition according to Claim 6, in which the photoprotective system is constituted of at least one hydrophilic, lipophilic or insoluble organic screening agent.

8. Composition according to Claim 6 or 7, in which the organic screening agents are chosen from anthranilates; cinnamic derivatives; dibenzoylmethane derivatives; salicylic derivatives; camphor derivatives; triazine derivatives; benzophenone derivatives; β-diphenylacrylate derivatives; benzotriazole derivatives; benzalmalonate derivatives; benzimidazole derivatives; imidazolines; bis-benzoazolyl derivatives; p-aminobenzoic acid (PABA) derivatives; methylenebis(hydroxyphenylbenzotriazole) derivatives; screening polymers and screening silicones; α-alkylstyrene-based dimers; 4,4-diaryl-butadienes, and mixtures thereof.

9. Composition according to Claim 8, in which the organic screening agents are chosen from:
Ethylhexyl methoxycinnamate,
Homosalate,
Ethylhexyl salicylate,
Butylmethoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate, 4-Methylbenzylidenecamphor,
Terephthalylidenedicamphorsulfonic acid,
Disodium phenyldibenzimidazoletetrasulfonate,
Ethylhexyl Triazone,
Bis-Ethylhexyloxyphenol Methoxyphenyl Triazine,
Diethylhexyl Butamido Triazone,
2,4,6-tris(Dineopentyl 4'-aminobenzalmalonate)-s-triazine,
2,4,6-tris(Diisobutyl 4'-aminobenzalmalonate)-s-triazine,
Methylenebis(benzotriazolyl)tetramethylbutylphenol, Drometrizole trisiloxane,
Polysilicone-15,
Dineopentyl 4'-methoxybenzalmalonate,
1,1-Dicarboxy(2,2'-dimethylpropyl)-4,4-diphenyl-butadiene,
2,4-Bis[5-1(dimethylpropyl)benzoxazol-2-yl(4-phenyl)-imino]-6-(2-ethylhexyl)imino-1,3,5-triazine,
and mixtures thereof.

10. Composition according to Claim 6, in which the pigments or nanopigments are chosen from coated or uncoated metal oxides.

11. Composition according to Claim 10, in which the mineral agent(s) for screening out UV radiation is (are) chosen from coated or uncoated titanium oxide, iron oxide, zinc oxide, zirconium oxide or cerium oxide nanopigments.

12. Composition according to any one of Claims 1 to 11, in which the photoprotective system is present in a content ranging from 0.1% to 40% by weight and preferably from 5% to 25% by weight relative to the total weight of the composition.

13. Composition according to any one of Claims 1 to 12, **characterized in that** the composition also contains at least one self-tanning agent.

14. Composition according to any one of Claims 1 to 13, in which the composition also contains at least one cosmetic adjuvant chosen from fatty substances, organic solvents, ionic or nonionic, hydrophilic or lipophilic thickeners, demulcents, humectants, opacifiers, stabilizers, emollients, silicones, antifoams, fragrances, preserving agents, anionic, cationic, nonionic, zwitterionic or amphoteric surfactants, active agents, fillers, polymers, propellants, basifying or acidifying agents or any other ingredient customarily used in the cosmetics and/or dermatological field.

15. Composition according to any one of Claims 1 to 14, **characterized in that** it is in particular in the form of a simple or complex emulsion, of a cream gel; of an aqueous gel; of a lotion, or is packaged as an aerosol in the form of a foam or a spray, or packaged as an atomizer.

16. Composition according to any one of Claims 1 to 15, **characterized in that** it is in the form of an oil-in-water or water-in-oil emulsion.

17. Composition according to Claim 16, containing at least one isophthalic acid or sulfoisophthalic acid polymer.

18. Composition according to Claim 17, in which said isophthalic acid or sulfoisophthalic acid polymer is Polyester-5.

19. Composition according to any one of the preceding claims, **characterized in that** it is in a vaporizable form.

20. Pressurization device comprising at least
(A) one reservoir containing at least one vaporizable fluid composition as defined in any one of Claims 1 to 19,
and (B) means for placing said composition under pressure.

21. Device according to Claim 20, **characterized in that** it is a non-aerosol pump.

22. Device according to either one of Claims 20 and 21, **characterized in that** it is an aerosol container or an aerosol pump.

23. Device according to any one of Claims 20 to 22, **characterized in that** it is a two-compartment aerosol container or aerosol pump.

24. Use of an ester-terminated poly(ester amide) polymer as defined in the preceding claims in a composition comprising, in a cosmetically acceptable aqueous support, at least one photoprotective system capable of screening out UV radiation as defined in one of the preceding claims, for the purpose of increasing the sun protection factor (SPF).
